# EUROPEAN PATENT SPECIFICATION

(11) **EP 2 180 047 B1**
(45) Date of publication and mention of the grant of the patent: **05.12.2012**
(21) Application number: 09012587.3
(22) Date of filing: 05.10.2009
(51) Int. Cl.: C12N 9/14, G01N 33/487, G01N 33/68

(54) **Biorhythm information acquisition method**
Verfahren zur Erfassung von Biorhythmusinformationen
Procédé d'acquisition d'informations de biorythme

(30) Priority: 07.10.2008 JP 2008260430
(43) Date of publication of application: 28.04.2010
(73) Proprietor: Sony Corporation, Tokyo 108-0075 (JP)
(72) Inventor: Hayakawa, Tomohiro, Tokyo 108-0075 (JP); Yamamoto, Takuro, Tokyo 108-0075 (JP); Nakagawa, Kazuhiro, Tokyo 108-0075 (JP); Soma, Haruhiko, Tokyo 108-0075 (JP); Yamashita, Shiko, Tokyo 108-0075 (JP); Wang, Fan, Tokyo 108-0075 (JP); Yasuda, Akio, Tokyo 108-0075 (JP)
(74) Representative: Müller - Hoffmann & Partner

(56) References cited:
- HORWITZ B L ET AL: "Diurnal profiles of tear lysozyme and gamma A globulin" ANNALS OF OPHTHALMOLOGY, ALTIER AND MAYNARD COMMUNICATIONS, RIDGEFIELD, CT, US, vol. 10, no. 1, 1 January 1978 (1978-01-01), pages 75-80, XP009130826 ISSN: 0003-4886
- RANTONEN P J ET AL: "Correlations between total protein, lysozyme, immunoglobulins, amylase, and albumin in stimulated whole saliva during daytime" ACTA ODONTOLOGICA SCANDINAVICA, OSLO, NO, vol. 58, no. 4, 1 August 2000 (2000-08-01) , pages 160-165, XP009130828 ISSN: 0001-6357
- DATABASE MEDLINE [Online] US NATIONAL LIBRARY OF MEDICINE (NLM), BETHESDA, MD, US; 1978, WAGNER V ET AL: "Levels of secretory immunoglobulin A, lysozyme (muramidase) and albumin in healthy adults of different ages." XP002573300 Database accession no. NLM570992
- NG V ET AL: "Variability of tear protein levels in normal young adults: between-day variation." GRAEFE'S ARCHIVE FOR CLINICAL AND EXPERIMENTAL OPHTHALMOLOGY = ALBRECHT VON GRAEFES ARCHIV FÜR KLINISCHE UND EXPERIMENTELLE OPHTHALMOLOGIE NOV 2000, vol. 238, no. 11, November 2000 (2000-11), pages 892-899, XP002573301 ISSN: 0721-832X
- SACK R A ET AL: "Diurnal tear cycle: evidence for a nocturnal inflammatory constitutive tear fluid." INVESTIGATIVE OPHTHALMOLOGY & VISUAL SCIENCE MAR 1992, vol. 33, no. 3, March 1992 (1992-03), pages 626-640, XP002573302 ISSN: 0146-0404
- WAGNER V ET AL: "Seasonal and sex-related changes in the levels of immunoglobulins and lysozyme in a semicohort of children during a three-year period" JOURNAL OF HYGIENE, EPIDEMIOLOGY, MICROBIOLOGY AND IMMUNOLOGY, AVICENUM, PRAGUE, CS, vol. 26, no. 2, 1 January 1982 (1982-01-01), pages 187-203, XP009130899 ISSN: 0022-1732
- RUDNEY J D ET AL: "Longitudinal study of relations between human salivary antimicrobial proteins and measures of dental plaque accumulation and composition" ARCHIVES OF ORAL BIOLOGY, PERGAMON PRESS, OXFORD, GB, vol. 38, no. 5, 1 May 1993 (1993-05-01), pages 377-386, XP026167236 ISSN: 0003-9969 [retrieved on 1993-05-01]
- RICHTER J ET AL: "Circadian changes of the SIgA, lysozyme, albumin and copper content of saliva" CZECH. MED, XX, XX, vol. 3, no. 4, 1 January 1980 (1980-01-01) , pages 249-254, XP009130827 ISSN: 0139-9179
- DATABASE MEDLINE [Online] US NATIONAL LIBRARY OF MEDICINE (NLM), BETHESDA, MD, US; 1989, WAGNER V ET AL: "Lack of correlation between serum and salivary concentration levels of immunoglobulin A and lysozyme (muramidase)." XP002573303 Database accession no. NLM2809190
- RUDNEY J D ET AL: "Correlations between human salivary levels of lysozyme, lactoferrin, salivary peroxidase and secretory immunoglobulin A with different stimulatory states and over time" ARCHIVES OF ORAL BIOLOGY, PERGAMON PRESS, OXFORD, GB, vol. 30, no. 11-12, 1 January 1985 (1985-01-01), pages 765-771, XP026154399 ISSN: 0003-9969 [retrieved on 1985-01-01]
- VASILEV N V ET AL: "Adaptation and nonspecific mechanisms of immunity" HUMAN PHYSIOLOGY,, vol. 4, no. 5, 1 January 1978 (1978-01-01) , pages 691-697, XP009130701 ISSN: 0362-1197
- UEDA HIROKI R ET AL: "Molecular-timetable methods for detection of body time and rhythm disorders from single-time-point genome-wide expression profiles.", PROCEEDINGS OF THE NATIONAL ACADEMY OF SCIENCES OF THE UNITED STATES OF AMERICA 3 AUG 2004 LNKD- PUBMED:15273285, vol. 101, no. 31, 3 August 2004 (2004-08-03), pages 11227-11232, ISSN: 0027-8424

## Description

### BACKGROUND OF THE INVENTION

### 1. Field of the Invention

The present invention relates to a method of acquiring information on a biorhythm of a bion. More particularly, the invention relates to a method of acquiring information on a circadian rhythm on the basis of variations with time of the quantities of a physiologically active substance in tear and saliva.

### 2. Description of the Related Art

It is known that various biophenomena in a bion each show a "periodic rhythm" which vibrates self-supportingly. The periodic rhythm is called "biorhythm." Especially, a "circadian rhythm" having a period of about one day controls widely the rhythms observed in biophenomena such as the sleep-wake rhythm and diurnal variation rhythms of body temperature, blood pressure and the quantities of hormones secreted. In addition, the circadian rhythm is known to relate to the levels of mental and physical activities, capacity for locomotion, drug sensitivity, and so on.

The biorhythm is controlled by a gene group called "clock gene." The clock gene (which may hereinafter be referred to also as "clock molecule") functions as a "biological clock" by autonomously periodically varying (vibrating) the expression, activity, localized presence or the like thereof.

It has been clarified that gene polymorphism and gene mutation of the clock molecule would be critical causes of cancer, diabetes, vascular diseases, altered-nerve diseases and the like. In recent years, furthermore, it has been pointed out that a gene polymorphism or mutation of the clock molecule relates also to the crisis of mental disorders such as bipolar disorder and depression. For curing these diseases, it has been attempted to reset, by irradiation with light, the biological clock having been brought out of order due to a gene polymorphism or mutation of the clock molecule.

On the other hand, the biorhythm is not only autonomously controlled by the biological clock but also is under restrictions by social life. For instance, in relation to the sleep-wake rhythm, a change in the bedtime or the rising time in daily life may cause a rhythm shift (phase shift) between the "going-to-bed and rising cycle in real life" and the "sleep-wake rhythm based on the biological clock." Such a shift in biorhythm is considered to induce the so-called "jet lag" or somnipathy and, further, to cause the above-mentioned mental disorders.

Furthermore, an attempt to maximize the effect of pharmacotherapy by utilizing the biorhythm has begun. Due to the amount of expression of a molecule serving as a target of a drug (drug target molecule) and the circadian rhythm of the activity of an enzyme which metabolizes the drug (drug-metabolizing enzyme), the therapeutic effect of the drug is also considered to show a diurnal variation. Taking this into account, there has been proposed a way of thinking called "chronotherapy" in which maximization of a therapeutic effect is contrived by determining an optimal medication time for each drug.

Besides, in a more familiar example, the acting time for bringing out personal ability to the full in learning or training and the eating time for a person to get fat with difficulty (or with ease) have come to be investigated utilizing the circadian rhythms of mental and physical activities and capacity for locomotion.

From the foregoing, it is considered that to accurately know the biorhythm based on a biological clock is very useful for prevention of various diseases, for improvement of poor physical condition such as jet lag, for realization of chronotherapy, for exhibition of personal ability, for a diet, and so on.

PCT Patent Publication No. WO 2004/012128 (hereinafter referred to as Patent Document 1) discloses, at least, a method for estimating a biological time on the basis of gene expression product amount measurement data on a standard specimen collected from a bion. In the biological clock estimation method, a molecular clock table for estimation of a biological clock is formed based on the expression amount of a gene expression product (specifically, mRNA (messenger ribonucleic acid)). Incidentally, Patent Document 1 does not describe a specific tissue (or cell) to be collected or a specific gene to be measured.

Japanese Patent Laid-open No. Hei 6-189914 (hereinafter referred to as Patent Document 2) describes a biorhythm curve measuring apparatus for measuring a biorhythm curve from measured values of deep body temperature of a human being. In the biorhythm curve measuring apparatus, a contrivance is made such that a true biorhythm curve can be measured through removing influences of disturbances (external influences). Incidentally, Patent Document 2 shows rectal temperature or tympanic temperature as a specific example of the deep body temperature, and describes that the rectal temperature is particularly preferable.

Horowitz B.L. et al., (Annals. Ophthalmology 10 (1978) pages 78 - 80) discloses the determination of tear lysozyme and gamma A globulin concentrations during 24 hour period wherein tear lysozyme was significantly elevated between the 9 and 12 AM periods and reduced during the sleep periods.

Rantonen P.J. et al. (Acta Odontologica Scandinavica 58 (2000) pages 160 - 1658) relates to the correlation between lysozyme and Immunoglobulin in saliva during daytime.

Richter J. et al. (Czech Med. 3 (1980) pages 249 - 254) relates to circadian changes of the secretory Immunoglobulin A, lysozyme in saliva.

### SUMMARY OF THE INVENTION

The biological clock estimation method disclosed in Patent Document 1 is a method based on the expression amount of mRNA which is a standard specimen collected from a bion. While Patent Document 1 does not describe a specific tissue (or cell) to be collected or a specific gene to be measured, a method of examining the expression of a clock gene in leukocyte has been widely adopted as a simple method. According to this method, however, drawing blood is indispensable and, hence, the method involves a physical pain in the subject.

Furthermore, it is necessary for the measuring person to conduct an operation of separating the leukocyte from the blood drawn, an operation of extracting mRNA from the leukocyte, analysis of the expression of the clock gene mRNA and the like, which has been the reason why it takes much time and labor to carry out the method. In order to extract mRNA from an organism specimen and determine the mRNA, in general, an intricate operation is demanded for preventing the mRNA from being decomposed. Especially, if decomposition of mRNA occurs in dealing with a very small amount of organism specimen, it would be impossible to obtain stable measurement results.

The biorhythm curve measuring apparatus disclosed in Patent Document 2 is an apparatus for measuring particularly the rectal temperature. Measurement of body temperature in the rectum, however, imposes a mental or physical pain on the subject and, therefore, the measuring person also gets a feeling of burden.

Thus, there is a need for a simple and minimally invasive method for acquiring information on a biorhythm of a bion . This is achieved by the subject watter of the independent claims.

According to an embodiment of the present invention, there is provided a method of acquiring information on a biorhythm on the basis of a variation with time of the quantity of a physiologically active substance in tear and/or saliva.

In this method, the physiologically active substance may be a secretory IgA (Immunoglobulin A) antibody, and the information can be acquired based on a variation with time of the quantity of the secretory IgA antibody in the tear and/or the saliva.

In the method, preferably, lysozyme is further used as the physiologically active substance, and the information is acquired based on a variation with time of the ratio of the quantity of the secretory IgA antibody to the quantity of lyzozyme ((quantity of secretory IgA)/(quantity of lysozyme)) in the tear and/or the saliva.

In the method, a shift in biorhythm, specifically, a shift between a sleep-wake rhythm in a circadian rhythm and a going-to-bed and rising cycle in real life, may be detected based on a phase shift between a variation curve showing a variation with time of the ratio of the quantity of the secretory IgA antibody in the tear to the quantity of lysozyme in the tear and a variation curve showing a variation with time of the ratio of the quantity of the secretory IgA antibody in the saliva to the quantity of lysozyme in the saliva.

In this method, preferably, a variation curve showing the variation with time is used as a molecular timetable for estimating the biorhythm.

Thus, in accordance with an embodiment of the present invention, a simple and minimally invasive method for acquiring information on a biorhythm of a bion can be provided.

### BRIEF DESCRIPTION OF THE DRAWINGS

FIG. 1 is a diagram for illustrating an example of a variation of sIgA/lysozyme with time;
FIGS. 2A and 2B are diagrams for illustrating a change in phase of a sIgA/lysozyme variation curve;
FIGS. 3A to 3F are diagrams showing measurement results of diurnal variations of sIgA/lysozyme in tear and saliva under the condition where the bedtime and the rising time are kept uniform, wherein FIG. 3A shows an average for five subjects, and FIGS. 3B to 3F show respective measurements for the subjects, and wherein time is taken on the axis of abscissas and sIgA/lyzozyme is taken on the axis of ordinates; and
FIGS. 4A to 4F are diagrams showing measurement results of diurnal variations of sIgA/lysozyme in tear and saliva under the condition where a factitious "jet lag" state is generated by changing the bedtime and the rising time (going-to-bed and rising cycle), wherein FIG. 4A shows an average for five subjects and FIGS. 4B to 4F show respective measurements for the subjects, and wherein time is taken on the axis of abscissas and sIgA/lysozyme is taken on the axis of ordinates.

### DETAILED DESCRIPTION OF THE PREFERRED EMBODIMENTS

### 1. Biorhythm Information Acquisition Method

In order to establish a method for acquiring information on a biorhythm of a bion easily and in a minimally invasive manner, the present inventors paid attention to tear and saliva which are biosamples capable of being collected in an extremely less invasive manner. It has been known that 60 or more physiologically active substances are contained in tear. The physiologically active substances include proteins which function as resisting power against infection, such as lactoferrin, lysozyme, secretory IgA, etc. and TSP (Tear Specific Prealbmin) which functions as a carrier for a liposoluble substance such as vitamin A. Besides, saliva contains a variety of physiologically active substances, for example, digestive enzymes such as amylase, etc., glycoproteins such as lactoferrin, mucin, etc., lipid, and vitamins.

The present inventors analyzed variations in the secretion amounts of these physiologically active substances contained in tear or saliva. As a result of the analyses, secretory IgA antibody was specified as a physiologically active substance of which the secretion amount shows a diurnal variation. Furthermore, it was found out that the diurnal variations in the quantities of the secretory IgA antibody secreted in tear and saliva show well-agreeing circadian rhythms.

Therefore, an embodiment of the present invention provides a method of acquiring information on a biorhythm on the basis of a variation with time of the quantity of secretory IgA antibody in tear or saliva. In the biorhythm information acquisition method, the bion to be dealt with not only includes the human being but also widely includes laboratory animals such as mice, rats and monkeys.

Referring more specifically to the secretory IgA antibody, an IgA antibody produced in plasma cells is bonded to a secretory piece produced in acinar cells or excretory duct cells of the lacrimal gland or the salivary gland to form the secretory IgA antibody, which is secreted into the tear or saliva. In the biorhythm information acquisition method pertaining to the present embodiment, the tear or saliva is collected from a bion, and the quantity of the secretory IgA antibody in the tear or saliva is measured, thereby to estimate a biorhythm of the bion.

### 2. Collection of Tear and Saliva

### (1) Collection of Tear

Collection of tear can be performed, for example, by collecting the tear accumulated at a lower eyelid (tear meniscus) upon reflex secretion (stimulated secretion). The reflex secretion can be induced, for example, by a stimulus as a result of keeping the eye open or by stimulating a nasal mucosa with a swab or the like.

The collection of tear can be carried out by a method of collecting the tear accumulated upon basal secretion or reflex secretion or by the eye flush method. In the case of the tear supplied by basal secretion, however, the collection amount is as small as about 1 to 2 µl, and the collection takes time. Besides, the collection of the tear supplied by reflex secretion involves a heavy burden on the subject. The eye flush method is a method in which a fixed amount of physiological saline or the like as eyewash is applied to the subject's eye and thereafter the tear is collected together with the physiological saline or the like. Although the tear is diluted with the physiological saline or the like in the eye flush method, the method is advantageous in that the tear component can be collected speedily and that the burden on the subject is light. In the present invention, the secretory IgA antibody is determined in terms of the ratio of its quantity to the quantity of lysozyme, as described later. Basically, therefore, the dilution of the tear does not matter. Accordingly, the eye flush method is suitable for use as a tear collection method in the present invention.

The diluted tear accumulated at the tear meniscus is collected by use of a micropipette or the like in such a manner as not to touch the eyeball. For the micropipette, a sterilized disposable chip can be used. Particularly, it is desirable to use a disposable chip in which a soft silicone tube is attached to a tapered tip thereof and which is ordinarily used for sample loading to an electrophoretic gel.

The diluted tear accumulated at the tear meniscus may be collected by use of a filter paper. In the case where a filter paper is used, however, it takes time to collect the tear, and a work for extracting a physiologically active substance from the filter paper after the collection is needed. Therefore, the method in which the micropipette or the like is used is desirable, since the method enables speedy and easy collection of tear.

The tear is collected by applying a preset amount of physiological saline as eyewash to the subject's eye. In the case where the determination of the physiologically active substance is not performed immediately after the collection of tear, it is desirable to preserve the collected tear by cryopreservation.

### (2) Collection of Saliva

Saliva can be collected by impregnating cotton with the saliva, setting the cotton in a tube, and putting the tube with the cotton therein to centrifugation. This operation can be carried out by use of a saliva collection implement or a saliva collection tube which is commercially available.

### 3. Determination of Physiologically Active Substance

Determination of a physiologically active substance can be carried out by a known method using, for example, a commercially available ELISA kit or a bioanalyzer produced by Agilent Technologies (see "Tear analysis and lens-tear interactions. Part I. Protein fingerprinting with microfluidic technology." Contact Lens & Anterior Eye, 2007, Vol. 30, No. 163). For the determination of a physiologically active substance, it is desirable to use tear or saliva supernatant from which impurities and mucin have been removed by centrifugation after collection or after thawing.

Here, the present inventors found out that by simultaneously determining also lysozyme which is one of the physiologically active substances contained in tear and saliva (see "Protein levels in nonstimulated and stimulated tears of normal human subjects." Investigative Ophthalmology & Visual Science, 1990, Vol. 31, No. 1119), the biorhythm can be estimated more accurately.

The concentrations of the physiologically active substances in tear and saliva are influenced by technical factors in collecting the tear and the saliva. Specifically, for example, in the case where tear supplied by basal secretion is collected, the collection amount is as small as about 1 to 2 µl, so that the concentrations of the physiologically active substances in the tear can largely vary due to slight evaporation of a tear component during the collecting operation. Besides, in collecting tear by the eye flush method, the concentrations of the physiologically active substances in the tear thus collected can vary depending on the amount of physiological saline or the like applied as eyewash to the subject's eye.

Such variations in the concentrations of the physiologically active substances due to the technical factors cause errors in the measured values of the quantities of the physiologically active substances. Therefore, for avoiding variations in the concentrations of the physiologically active substances due to the technical factors, it is desirable to adopt as an indicator a physiologically active substance which is contained in the tear and the saliva in constantly stably fixed quantities, and to compensate for the influences of evaporation of the tear or dilution of the tear with physiological saline or the like during collection of the tear.

The present inventors, during their analysis of variations in secretion quantities of these physiologically active substances contained in tear and saliva, identified lysozyme as a substance which is contained in the tear and the saliva in constantly stably fixed quantities. Such a substance which is contained in a sample in a constantly stably fixed quantity is generally called an "inner standard" and is used for correction of measurement errors.

Specifically, in the biorhythm information acquisition method pertaining to the present embodiment, the quantities of secretory IgA antibody and lysozyme contained in tear and saliva are measured, and the quantity of the secretory IgA antibody is corrected based on the quantity of lysozyme, to thereby acquire information on a biorhythm. More specifically, the measured value of the quantity of the secretory IgA antibody is divided by the measured value of the quantity of lysozyme, whereby a variation with time of the quantity of the secretory IgA antibody is determined in terms of the ratio of the quantity of the secretory IgA antibody to the quantity of lysozyme. This makes it possible to avoid the influences of evaporation of tear and dilution of the tear with physiological saline or the like at the time of collection of the tear, to correct the measurement errors in the quantity of the physiologically active substance due to the technical factors, and to estimate the biorhythm accurately.

In this manner, in the biorhythm information acquisition method pertaining to the present embodiment, tear and/or saliva which is easy to collect is used, whereby mental and physical burdens on the subject can be made extremely light, as compared with the methods according to the related art. In addition, since proteins stabler than mRNA are adopted as substances to be measured, the biorhythm can be easily estimated without need for intricate operations.

### 4. Molecular Timetable for Estimation of Biorhythm

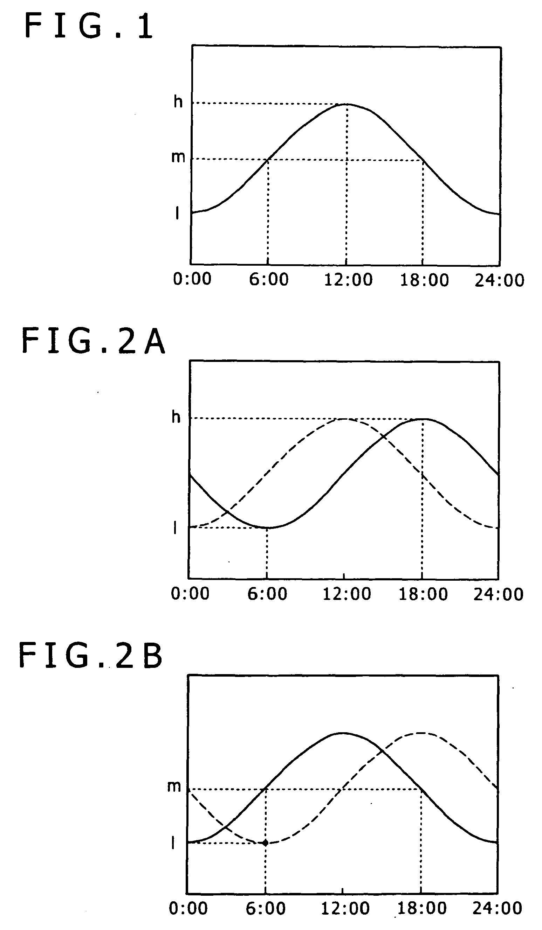

FIG. 1 is a diagram showing a variation with time of a value obtained by dividing the quantity of secretory IgA antibody contained in tear or saliva by the quantity of lysozyme contained in the tear or saliva (the value will hereinafter be described as "sIgA/lysozyme"). FIG. 1 shows an example of a variation curve obtained by measuring sIgA/lysozyme by the above-mentioned method at predetermined times in one day and plotting the measured values. In the diagram, time is taken on the axis of abscissas, and sIgA/lysozyme is taken on the axis of ordinates. FIG. 1 shows a case where a minimum (1) of sIgA/lysozyme is measured at 0:00 and a maximum (h) of sIgA/lysozyme is measured at 12:00.

The variation curve can be obtained, by inspection, from the plot of sIgA/lysozyme measured at each of the times in a day. Besides, for obtaining an accurate variation curve, use may be made of a period calculation method such as an autocorrelation method (correlogram), a power spectrum method, a cosinor method, and a periodogram method.

The ratio sIgA/lysozyme undergoes a diurnal variation, and shows a circadian rhythm as shown in FIG. 1. Therefore, information on a biorhythm of a subject can be obtained based on the variation of sIgA/lysozyme with time. Then, by utilizing the variation curve of sIgA/lysozyme as a "molecular timetable," the biorhythm of the subject can be estimated.

Specifically, for example, in regard of a subject known to have the variation curve (hereinafter, this term will be used in the same meaning as the "molecular timetable") shown in FIG. 1, it is assumed that sIgA/lysozyme measured at a predetermined time is h. In this case, based on the variation curve (molecular timetable) in FIG. 1, the circadian rhythm of the subject can be estimated to be at 12:00. Besides, in the case where sIgA/lysozyme is 1, the circadian rhythm of the subject can be estimated to be at 0:00, and in the case where sIgA/lysozyme is m, the circadian rhythm of the subject can be estimated to be at 6:00 or 18:00.

In addition, in regard of the same subject, it is assumed for example that values of sIgA/lysozyme measured twice at a time interval of three hours are p and q, respectively. In this instance, where q is higher than p (p < q), the circadian rhythm of the subject can be estimated to be in the morning (0:00 to 12:00) corresponding to a rising phase of sIgA/lysozyme. On the contrary, where q is lower than p (q < p), the circadian rhythm of the subject can be estimated to be in the afternoon (12:00 to 24:00). Further, by obtaining the coefficient of variation (q/p) of p and q and collating it with the inclination of a tangent to the variation curve, the time in the circadian rhythm can be estimated more accurately.

Now, a method of detecting a shift in the biorhythm of a subject on the basis of a variation of the variation curve will be described below.

The variation curve can be decided in shape by its maximum (or minimum), the time at which the maximum (or minimum) is observed, the inclination of the curve from the maximum to the minimum (or from the minimum to the maximum), and so on. In the present invention, the shape of the variation curve is referred to as "phase." Besides, the shape of the circadian rhythm of a subject specified by the variation curve (molecular timetable) is also referred to as "phase."

Specifically, the variation curve (molecular timetable) shown in FIG. 1 has a shape, or "phase," that is decided by the minimum 1, the maximum h, the minimum observation time 0:00, and the maximum observation time 12:00.

FIGS. 2A and 2B are diagrams showing a change in phase of a variation curve. In FIG. 2A, the variation curve indicated by dotted line is the curve shown in FIG. 1 (hereinafter, this curve may be referred to also as "molecular timetable 1"), and the solid line represents an example of a variation curve obtained by the same measurement as that in FIG. 1, in a different measurement day (hereinafter, this curve may be referred to also as "molecular timetable 2"). In the diagrams, time is taken on the axis of abscissas and sIgA/lysozyme is taken on the axis of ordinates.

The molecular timetable 1 has a phase in which the observation time of the minimum (1) is 0:00, and the observation time of the maximum (h) is 12:00. In contrast, in the molecular timetable 2, the phase is so changed that the observation time of the minimum (1) is 6:00 and the observation time of the maximum (h) is 18:00.

It can be estimated that a phase shift of the variation curve of the subject is generated between a time when the molecular timetable 1 is formed and a time when the molecular timetable 2 is formed. Specifically, the biorhythm of the subject at the time when the molecular timetable 2 is formed is getting six hours behind the time when the molecular timetable 1 is formed (or getting 18 hours ahead).

In this manner, formation of the molecular timetable is conducted a plurality of times for the same subject and the molecular timetables thus formed are compared with each other, whereby a phase shift in the biorhythm of the subject can be detected.

In addition, as another method for detecting a phase shift in a biorhythm, the following method may also be contemplated.

FIG. 2B is a diagram obtained from FIG. 2A by changing the molecular timetable 1 (see FIG. 1, also) from dotted line to solid line and changing the molecular timetable 2 from solid line to dotted line.

As has been described above, in regard of a subject known to have the variation curve (molecular timetable) shown in FIG. 1, in the case where sIgA/lysozyme measured at a predetermined time is m it can be estimated that the circadian rhythm of the subject is at 6:00 or 18:00.

Here, for the same subject, it is assumed that the value of sIgA/lysozyme obtained by measurement at 6:00 on a different measurement day has changed from m to 1 (see the circular mark in FIG. 2B). In this case, the circadian rhythm of the subject can be estimated to have changed into a circadian rhythm shown in the molecular timetable 2 by getting six hours behind the original (or by getting 18 hours ahead).

In this manner, sIgA/lysozyme at a predetermined time is compared with a preliminarily prepared molecular timetable, whereby a phase shift in the biorhythm of a subject can be detected more easily.

Thus, according to the biorhythm information acquisition method pertaining to the present embodiment, a variation curve showing a variation with time of sIgA/lysozyme in tear or saliva is utilized as a molecular timetable, whereby it is possible to estimate the biorhythm intrinsic of the subject and to detect a phase shift in the biorhythm.

Here, as will be shown in Example below, the present inventors found out that the diurnal variations of sIgA/lysozyme in tear and saliva show well-agreeing circadian rhythms. Therefore, in the biorhythm information acquisition method pertaining to the present embodiment, the information on the biorhythm as above-mentioned can be acquired based on the variation with time of sIgA/lysozyme in either of tear and saliva. In addition, more accurate information can be acquired based on time variations in values of sIgA/lysozyme in both tear and saliva. Furthermore, detailed information on biorhythm can also be obtained, by detecting a phase shift based on variation curves showing time variations of sIgA/lysozyme in both tear and saliva.

### 5. Detection of Shift between Sleep-wake Rhythm and Going-to-bed and Rising Cycle

Specifically, in the biorhythm information acquisition method pertaining to the present embodiment, a shift between a "sleep-wake rhythm" in a circadian rhythm and a "going-to-bed and rising cycle" in real life can be detected, based on a phase shift between a variation curve showing the variation with time of sIgA/lysozyme in tear and a variation curve showing the variation with time of sIgA/lysozyme in saliva.

As has been described above, the "sleep-wake rhythm" in a circadian rhythm is not only controlled autonomously by the biological clock but also undergoes restrictions by the social life. Therefore, due to changes in the bedtime and the rising time in daily life, a shift in rhythm (a shift in phase) may be generated between the "going-to-bed and rising cycle" in real life and the "sleep-wake rhythm" by the biological clock.

The present inventors found out that when a change is given to the going-to-bed and rising cycle as shown in Example below, the variation curve of sIgA/lysozyme in tear is changed in phase in sharp response to the change in the going-to-bed and rising cycle. Besides, on the other hand, it was clarified by the present inventors that the variation curve of sIgA/lysozyme in saliva is not changed in phase immediately upon the change in the going-to-bed and rising cycle but is changed in phase over a few days so that its phase gradually conforms to the phase of the variation curve of sIgA/lysozyme in tear.

This fact can be understood to show that a phase shift between the variation curve indicative of the variation with time of sIgA/lysozyme in tear and the variation curve indicative of the variation with time of sIgA/lysozyme in saliva represents a shift generated between the "going-to-bed and rising cycle" in real life and the "sleep-wake rhythm" inherent to the circadian rhythm. In addition, this shift between the "going-to-bed and rising cycle" and the "sleep-wake rhythm" is considered to be dissolved by gradual conformation of the phase of the variation curve of sIgA/lysozyme in saliva to the phase of the variation curve of sIgA/lysozyme in tear, which is precedently changed in phase, through the process in which the "sleep-wake rhythm" by the biological clock is gradually adapted to the new "going-to-bed and rising cycle" after the change.

Therefore, it can be considered to be possible to detect the generation of a shift between the "sleep-wake rhythm" in the circadian rhythm and the "going-to-bed and rising cycle" in real life, based on the phase shift between the variation curve indicative of the variation with time of sIgA/lysozyme in tear and the variation curve indicative of the variation with time of sIgA/lysozyme in saliva. Example

Under the following conditions, diurnal variations of sIgA/lysozyme in tear and saliva were measured, and it was tried to estimate a biorhythm and to detect a shift between a sleep-wake rhythm and a going-to-bed and rising cycle.

### (1) Subject

Tear and saliva samples were taken from five male subjects who were 33 to 41 in age. The sampling was performed at a fixed time interval, and the sampling during when the subjects were sleeping was carried out by momentarily waking up the subjects. The sampling was conducted in two patterns, specifically, under a condition where the bedtime and the rising time during the experimental period were kept uniform and under a condition where the bedtime and the rising time were changed during the experimental period. Incidentally, during the experimental period, the subjects were made to give up taking of coffee, alcohol and the like which could influence their autonomic nervous activities.

### (2) Tear and Saliva Collection Methods

Collection of tear was conducted by the eye flush method. Specifically, first, 30 µl of physiological saline (room temperature) as eyewash was applied to an eye of each subject. The eyewash application was carried out by use of a commercially available eyedrop vessel, and to the center of the eyeball. The diluted tear accumulated at the lower eyelid (tear meniscus) upon the eyewash application was swiftly collected by use of a microchip which is ordinarily used for sample loading to an electrophoretic gel. The microchip having a tapered tip and having been sterilized was used with a soft silicone tube attached to the tip thereof. The diluted tear thus collected was put in a 1.5 ml Eppendorf tube, and was preserved in a freezer at -30°C until determination of secretory IgA. Besides, collection of saliva was performed by use of a salivette (produced by Sarstedt AG & Co.) which is a saliva collection implement.

### (3) Measurement of Quantity of Secretory IgA Antibody

The quantities of secretory IgA antibody and lysozyme in tear and saliva were determined by use of a bioanalyzer (produced by Agilent Technologies Inc.). By use of an exclusive-use microchannel chip (Protein 230), an electrophoretic profile (chromatogram) of proteins was obtained from 4 µl of tear or saliva. By use of the software (Expert 2100) attached to the analyzer, the quantity of each of the proteins detected as peaks in the chromatogram obtained was determined.

### 1. Estimation of Biorhythm Based on Variations with Time of sIgA/lysozyme in Tear and Saliva

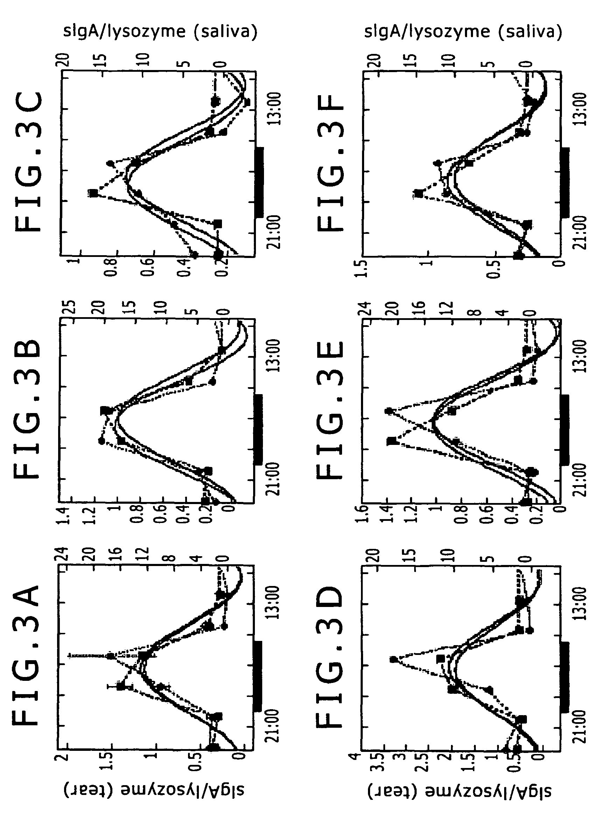

FIGS. 3A to 3F show the measurement results of diurnal variations of sIgA/lysozyme in tear and saliva under the condition where the bedtime and the rising time were kept uniform. The figures show the measurement results of IgA/lysozyme in a day (the thick bar along the axis of abscissas in each of the figures indicates the sleeping time zone). FIG. 3A shows an average for the five subjects, and FIGS. 3B to 3F show the measured values for the subjects, respectively. In the figures, time is taken on the axis of abscissas and sIgA/lysozyme is taken on the axis of ordinates.

In each of FIGS. 3A to 3F, the sIgA/lysozyme in tear which is indicated by the dotted line connecting the circular plots and the sIgA/lysozyme in saliva which is indicated by the dotted line connecting the square plots show periodic diurnal variations, and it was confirmed that the diurnal variations each show a circadian rhythm. Besides, from the variation curve (see the solid line in each figure) obtained by fitting these plots to a cosine function having a period of 24 hours, it was clarified that the diurnal variations of sIgA/lysozyme in tear and saliva show well-agreeing circadian rhythms.

### 2. Detection of Shift between Sleep-wake Rhythm and Going-to-bed and Rising Cycle

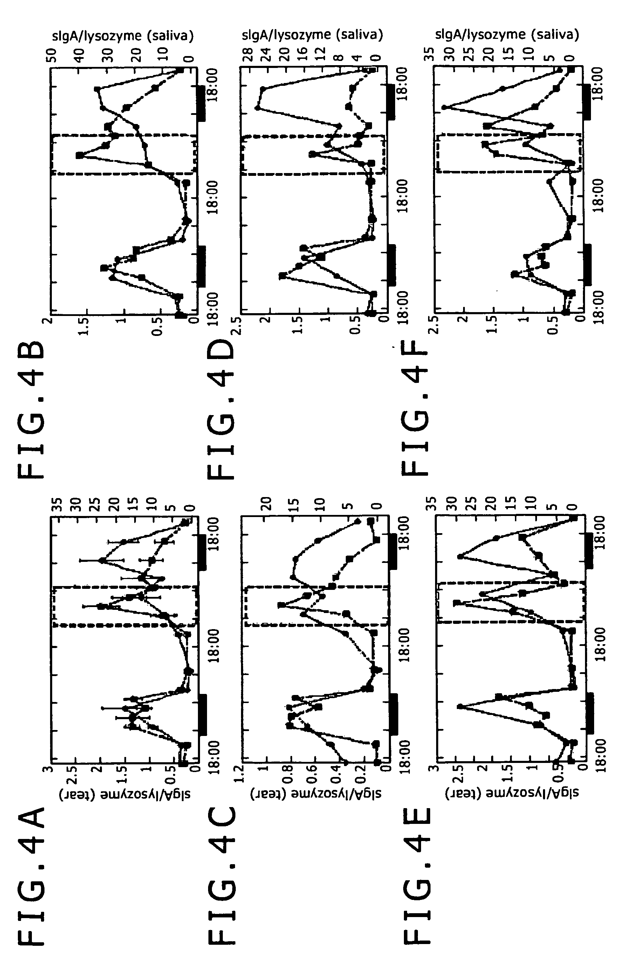

FIGS. 4A to 4F show the measurement results of diurnal variations of sIgA/lysozyme in tear and saliva under the condition where a factitious "jet lag" state was generated by changing the bedtime and the rising time (going-to-bed and rising cycle). The figures show the results of measurement of sIgA/lysozyme carried out on the day before the change of the bedtime and rising time and on two days when the bedtime was set 11 hours behind by sitting up all night (in the figures, the thick bars along the axis of abscissas represent the sleeping time zones). FIG. 4A shows an average for the five subjects, and FIGS. 4B to 4F show the measured values for the subjects, respectively. Incidentally, time (with the sleeping time zone being represented by the thick bar) is taken on the axis of abscissas, and sIgA/lysozyme is taken on the axis of ordinates.

On the day before the change of the bedtime and the rising time, the diurnal variations of sIgA/lysozyme in tear and saliva agreed well with each other. Upon evaluation of a peak time by fitting of a cosine curve having a period of 24 hours, the maximum (peak) was observed at around 4:30 in the sleeping time zone (23:00 to 7:30) for everyone of the five subjects.

Thereafter, when the bedtime was set 11 hours behind by sitting up all night so that the sleeping time zone (from the bedtime to the rising time) would be 10:00 to 17:30 and a factitious "jet lag" state would be generated, sIgA/lysozyme in saliva (square-plot dotted line) showed a maximum (peak) at around 3 to 5 o'clock in the original sleeping time zone (23:00 to 7:30, the time zone surrounded by broken line in each figure), in the same manner as before the change of the bedtime and the rising time. On the other hand, sIgA/lysozyme in tear (circular-plot dotted line) showed a maximum (peak) at around 13:30 to 17:30 in the new sleeping time zone (10:00 to 17:30), in response to the change of the bedtime and the rising time.

These results show that when a shift is generated between a "sleep-wake rhythm" inherent to a circadian rhythm and a "going-to-bed and rising cycle" in real life by generation of a factitious "jet lag" state, a phase shift may possibly be generated between the diurnal variation of sIgA/lysozyme in tear and the diurnal variation of sIgA/lysozyme in saliva.

In addition, the phase shift between the diurnal variation of sIgA/lysozyme in tear and that in saliva was gradually reduced so that also sIgA/lysozyme in saliva came to show a maximum (peak) at around 13:30 to 17:30 in the new sleeping time zone (10:00 to 17:30) on the 5th day from the day of the change of the bedtime and rising time.

From these results, it was considered that the phase shift between the diurnal variation of sIgA/lysozyme in tear and the diurnal variation of sIgA-lysozyme in saliva can be used as an indicator of a "jet lag" state generated between the "going-to-bed and rising cycle" in real life and the "sleep-wake rhythm" inherent to the circadian rhythm.

According to the biorhythm information acquisition method pertaining to the present embodiment, a variation curve indicative of a variation in sIgA/lysozyme with time is utilized as a molecular timetable, whereby a biorhythm intrinsic of each subject can be estimated easily and in a minimally invasive manner. This makes it possible for each individual to know his or her own intrinsic biorhythm, and makes it possible to set an optimum medication time, an optimum acting time and an optimum eating time. Therefore, the biorhythm information acquisition method pertaining to the present embodiment can be effectively used for realization of chronotherapy, for exhibition of one's own capacity and for a diet.

Furthermore, according to the biorhythm information acquisition method pertaining to the present embodiment, a phase shift in a biorhythm can be detected easily and in a minimally invasive manner. Therefore, the biorhythm information acquisition method can be effectively used for prevention of various diseases which might arise from a shift in biorhythm, and for improvement of a poor physical condition such as jet lag.

## Claims

1. A method of acquiring information on a circadian biorhythm on the basis of a measurement of a variation with time of the quantity of a secretory Immunoglobulin A antibody and lysozyme wherein the information is acquired based on a variation with time of the ratio of the quantity of the secretory Immunoglobulin A antibody to the quantity of lysozyme (quantity of secretory Immunoglobulin A/quantity of lysozyme) in the tear and the saliva.

2. The method according to claim 1, wherein a shift in the biorhythm is detected based on a phase shift between a variation curve showing a variation with time of the ratio of the quantity of the secretory Immunoglobulin A antibody in the tear to the quantity of lysozyme in the tear and a variation curve showing a variation with time of the ratio of the quantity of the secretory Immunoglobulin A antibody in the saliva to the quantity of lysozyme in the saliva.

3. The method according to claim 2, wherein a shift between a sleep-wake rhythm in a circadian rhythm and a going-to-bed and rising cycle in real life is detected as a shift in the biorhythm.

4. The method according to any of claims 1 to 3, wherein a variation curve showing the variation with time is used for estimating the biorhythm.

## Patentansprüche

1. Verfahren zum Erfassen von Informationen eines zirkadianen Biorhythmus auf der Basis einer Messung einer Variation der Menge eines sekretorischen Immunglobulin-A-Antikörpers und Lysozym über die Zeit, wobei die Information basierend auf einer Variation des Verhältnisses der Menge des sekretorischen Immunglobulin-A-Antikörpers und der Menge des Lysozyms (Menge des sekretorischen Immunglobulin-A/Menge des Lysozyms) in Tränen und Speichel über die Zeit erfasst wird.

2. Verfahren nach Anspruch 1, wobei eine Verschiebung des Biorhythmus nachgewiesen wird, basierend auf einer Phasenverschiebung zwischen einer Variationskurve, die eine Variation über die Zeit des Verhältnisses der Menge des sekretorischen Immunglobulin-A-Antikörpers in Tränen zur Menge des Lysozyms in Tränen zeigt, und einer Variationskurve, die eine Variation über die Zeit des Verhältnisses der Menge des sekretorischen Immunglobulin-A-Antikörpers im Speichel zur Menge des Lysozyms im Speichel zeigt.

3. Verfahren nach Anspruch 2, wobei eine Verschiebung zwischen dem Schlaf-Wach-Rhythmus in einem zirkadianen Rhythmus und ein Bettgeh - und Aufsteh-Zyklus im realen Leben als Verschiebung im Biorhythmus nachgewiesen wird.

4. Verfahren nach einem der Ansprüche 1 bis 3, wobei eine Variationskurve, die die Variation über die Zeit zeigt, zur Abschätzung des Biorhythmus eingesetzt wird.

## Revendications

1. Procédé d'acquisition d'informations sur un biorythme circadien sur la base de la mesure d'une variation avec le temps de la quantité d'un anticorps immunoglobuline A sécrétoire et de lysozyme où les informations sont acquises en se basant sur une variation avec le temps du rapport de la quantité de l'anticorps immunoglobuline A sécrétoire sur la quantité de lysozyme (quantité d'immunoglobuline A sécrétoire/quantité de lysozyme) dans les larmes et la salive.

2. Procédé selon la revendication 1, dans lequel un écart dans le biorythme est détecté en se basant sur un écart de phase entre une courbe de variation montrant une variation avec le temps du rapport de la quantité de l'anticorps immunoglobuline A sécrétoire dans les larmes sur la quantité de lysozyme dans les larmes et une courbe de variation montrant une variation avec le temps du rapport de la quantité de l'anticorps immunoglobuline A sécrétoire dans la salive sur la quantité de lysozyme dans la salive.

3. Procédé selon la revendication 2, dans lequel un écart entre un rythme veille-sommeil dans un rythme circadien et un cycle de coucher et lever dans la vie réelle est détecté comme un écart dans le biorythme.

4. Procédé selon l'une quelconque des revendications 1 à 3, dans lequel une courbe de variation montrant la variation avec le temps est utilisée pour estimer le biorythme.
